Europäisches Patentamt

European Patent Office

Office européen des brevets

(11) Publication number: **0 247 600 B1**

# EUROPEAN PATENT SPECIFICATION

(45) Date of publication of patent specification: **13.05.92**

(51) Int. Cl.⁵: **C12N 15/53**, C12N 1/20, C12P 21/02, //C12N9/02, (C12N1/20,C12R1:19)

(21) Application number: **87107740.0**

(22) Date of filing: **27.05.87**

(54) Benzene oxygenase gene.

(30) Priority: **28.05.86 JP 121263/86**

(43) Date of publication of application:
**02.12.87 Bulletin 87/49**

(45) Publication of the grant of the patent:
**13.05.92 Bulletin 92/20**

(84) Designated Contracting States:
**BE CH DE FR GB IT LI NL SE**

(56) References cited:
**EP-A- 0 088 602**
**EP-A- 0 098 137**

**PATENT ABSTRACTS OF JAPAN, vol. 10, no. 345 (C-386)[2401], 20th November 1986; & JP-A-61 149 081 (IDEMITSU KOSAN CO., LTD) 07-07-1986**

(73) Proprietor: **IDEMITSU KOSAN COMPANY LIMITED**
**1-1, Marunouchi 3-chome Chiyoda-ku Tokyo(JP)**

(72) Inventor: **Doi, Seiji**
**593-2, Kimitsuka**
**Ichihara-shi Chiba-ken(JP)**
Inventor: **Irie, Shinji**
**1218-2, Kamiizumi**
**Sodegaura-machi Kimitsu-gun Chiba-ken(JP)**

(74) Representative: **Vossius & Partner**
**Siebertstrasse 4 P.O. Box 86 07 67**
**W-8000 München 86(DE)**

**Description**

BACKGROUND OF THE INVENTION

1,2-Dihydroxy-cyclohexa-3,5-diene is of high utility value and has been used as a starting material for preparing engineering plastics (See: Japanese Patent Application Kokai Koho No. 71903/1983).

Only one method has been known for the production of 1,2-dihydroxy-cyclohexa-3,5-diene, which is described in the specification of Japanese Patent Application Kokai Koho No. 71891/1983.

In this known method, an aromatic compound is treated with a mutant of Pseudomonas putida, and is converted to 1,2-dihydroxy-cyclohexa-3,5-diene.

This method, however, has some disadvantages. It is difficult to handle the cells of Pseudomonas putida and a long time is required for the production of the desired compound.

There has been made no attempt either to isolate benzene oxygenase gene or to transform microorganisms with this genetic information.

The present inventors, after extensive research, have succeeded in isolating a DNA sequence that corresponds to a part of the gene which is contained in a microorganism capable of producing catechol from benzene or its derivative (See: Japanese Patent Application No. 68700/1986). At least gene DNA segments Nos. I, II, III and IV were found to be the base sequences, in combination, coding for an amino acid sequence of one particular enzyme protein. Said gene DNA is occasionally isolated in such a form that a DNA segment designated as V is contained together with the DNA segments Nos. I, II, III and IV.

In the further progress of the research, the present inventors have now succeeded in the isolation of a DNA sequence that contains at least DNA segments Nos. I, II and III localized therein, but is devoid of the segment No. IV,and encodes a polypeptide having benzene oxygenase activity. The present inventors have also succeeded in the preparation of a recombinant DNA consisting of said DNA sequence and a vector, and in the transformation of prokaryotic cells by the use of said recombinant DNA. The present inventors have found that the transformed prokaryotic cells produce 1,2-dihydroxy-cyclohexa-3,5-diene from benzene.

SUMMARY OF THE INVENTION

This invention relates to a DNA sequence (gene) coding for a polypeptide having benzene oxygenase activity, and to a prokaryotic cell transformed with said gene. This invention also relates to a process for the production of 1,2-dihydroxy-cyclohexa-3,5-diene by use of said transformed cells. The invention is defined in the claims.

BRIEF DESCRIPTION OF THE DRAWING

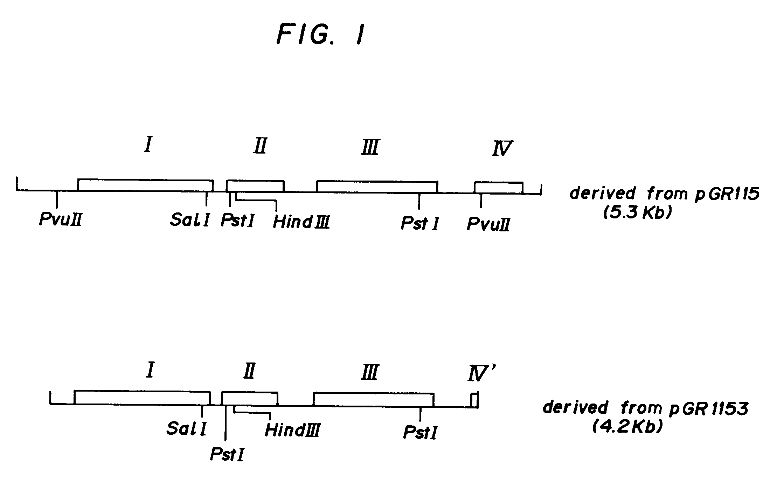

Fig. 1 shows a restriction enzyme cleavage map of DNA (nucleotide) segments contained in pGR115 and pGR1153, which are derived from a microorganism having benzene-oxidizing ability.

DETAILED DESCRIPTION OF THE INVENTION

According to the first aspect of this invention, there is, therefore, provided a DNA sequence encoding a polypeptide having benzene oxygenase activity which contains at least DNA segments Nos. I, II and III in combination. These sequences have the following formulae:

```
Segment No. I

    ATGA ATCAGACCGA CACATCACCT ATCAGGCTGC
    TACT TAGTCTGGCT GTGTAGTGGA TAGTCCGACG


    GCAGGAGCTG GAACACCAGC GAGATAGAAG CGCTCTTTGA
    CGTCCTCGAC CTTGTGGTCG CTCTATCTTC GCGAGAAACT


    CGAGCATGCC GGACGTATCG ATCCGCGCAT TTATACCGAT
    GCTCGTACGG CCTGCATAGC TAGGCGCGTA AATATGGCTA


    GAGGATCTGT ACCAACTCGA ACTGGAGCGT GTCTTCGCCC
    CTCCTAGACA TGGTTGAGCT TGACCTCGCA CAGAAGCGGG
```

3

```
GGTCCTGGCT  GCTGTTGGGG  CATGAAACCC  AGATTCGCAA
CCAGGACCGA  CGACAACCCC  GTACTTTGGG  TCTAAGCGTT


GCCGGGCGAT  TACATCACGA  CCTACATGGG  TGAAGACCCT
CGGCCCGCTA  ATGTAGTGCT  GGATGTACCC  ACTTCTGGGA


GTCGTGGTCG  TCCGGCAGAA  AGACGCCAGC  ATTGCCGTGT
CAGCACCAGC  AGGCCGTCTT  TCTGCGGTCG  TAACGGCACA


TCCTGAACCA  GTGCCGCCAC  CGTGGCATGC  GCATCTGCCG
AGGACTTGGT  CACGGCGGTG  GCACCGTACG  CGTAGACGGC


CGCGGATGCC  GGAAACGCGA  AGGCGTTCAC  TTGCAGCTAC
GCGCCTACGG  CCTTTGCGCT  TCCGCAAGTG  AACGTCGATG


CACGGGTGGG  CTTACGACAC  CGCCGGCAAT  CTTGTCAATG
GTGCCCACCC  GAATGCTGTG  GCGGCCGTTA  GAACAGTTAC


TGCCTTACGA  GGCCGAATCC  TTCGCGTGCC  TGAACAAGAA
ACGGAATGCT  CCGGCTTAGG  AAGCGCACGG  ACTTGTTCTT


GGAATGGAGC  CCGCTGAAGG  CCCGGGTAGA  AACCTACAAG
CCTTACCTCG  GGCGACTTCC  GGGCCCATCT  TTGGATGTTC


GGCCTGATTT  TCGCCAACTG  GGATGAGAAC  GCTGTAGACC
CCGGACTAAA  AGCGGTTGAC  CCTACTCTTG  CGACATCTGG


TCGACACGTA  TCTGGGCGAG  GCGAAGTTCT  ACATGGACCA
AGCTGTGCAT  AGACCCGCTC  CGCTTCAAGA  TGTACCTGGT


CATGCTCGAC  CGCACCGAGG  CCGGCACCGA  AGCGATCCCG
GTACGAGCTG  GCGTGGCTCC  GGCCGTGGCT  TCGCTAGGGC


GGCGTGCAGA  AGTGGGTCAT  TCCCTGTAAC  TGGAAATTCG
CCGCACGTCT  TCACCCAGTA  AGGGACATTG  ACCTTTAAGC


CCGCAGAGCA  GTTTTGCAGC  GACATGTACC  ATGCCGGGAC
GGCGTCTCGT  CAAAACGTCG  CTGTACATGG  TACGGCCCTG
```

4

```
GACCTCGCAT  CTGTCTGGCA  TCCTGGCAGG  CCTGCCAGAA
CTGGAGCGTA  GACAGACCGT  AGGACCGTCC  GGACGGTCTT


GACCTTGAAA  TGGCCGACCT  TGCTCCGCCG  ACAGTTGGCA
CTGGAACTTT  ACCGGCTGGA  ACGAGGCGGC  TGTCAACCGT


AGCAGTACCG  TGCGTCATGG  GGCGGACATG  GAAGTGGCTT
TCGTCATGGC  ACGCAGTACC  CCGCCTGTAC  CTTCACCGAA


CTATGTCGGC  GACCCCAATC  TGATGCTTGC  CATCATGGGG
GATACAGCCG  CTGGGGTTAG  ACTACGAACG  GTAGTACCCC


CCAAAGGTCA  CCAGCTACTG  GACCGAAGGC  CCCGCGTCGG
GGTTTCCAGT  GGTCGATGAC  CTGGCTTCCG  GGGCGCAGCC


AAAAGGCGGC  CGAACGTCTG  GGTAGCGTGG  AGCGCGGCTC
TTTTCCGCCG  GCTTGCAGAC  CCATCGCACC  TCGCGCCGAG


GAAACTCATG  GTCGAGCACA  TGACCGTCTT  CCCCACGTGT
CTTTGAGTAC  CAGCTCGTGT  ACTGGCAGAA  GGGGTGCACA


TCCTTCCTCC  CAGGTATCAA  TACGGTCCGG  ACATTGGCAT
AGGAAGGAGG  GTCCATAGTT  ATGCCAGGCC  TGTAACCGTA


CCGCGCGGGC  CGAACGAGGT  GAGGTATGGG  CGTTTACGGT
GGCGCGCCCG  GCTTGCTCCA  CTCCATACCC  GCAAATGCCA


GGTCGATGCT  GATGCTCCTG  ACGATATCAA  GGAAGAGTTC
CCAGCTACGA  CTACGAGGAC  TGCTATAGTT  CCTTCTCAAG


CGCGCCAGAC  TGCGCACCTT  CTCTCCGGTG  GCGTGTTCGA
GCGCGGTCTG  ACGCGTGGAA  GAGAGGCCAC  CGCACAAGCT


GCAGGACGAC  GGGGAGAACT  GGGTCGAGAT  CCAGCACATC
CGTCCTGCTG  CCCCTCTTGA  CCCAGCTCTA  GGTCGTGTAG


CTGCGAGGCC  ACAAGCCGGA  GCCGCCCTTT  CAATGCCGAG
GACGCTCCGG  TGTTCGGCCT  CGGCGGGAAA  GTTACGGCTC
```

```
ATGAGCATGG ACCAGACCGT CGACAACGAC CCGGTTTACC
TACTCGTACC TGGTCTGGCA GCTGTTGCTG GGCCAAATGG


CCGGGCGGAT CAGCAACAAC GTCTACAGCG AGGAAGCTGC
GGCCCGCCTA GTCGTTGTTG CAGATGTCGC TCCTTCGACG


CCGCGGGCTC TATGCCCATT GGCTGCGGAT GATGACATCC
GGCGCCCGAG ATACGGGTAA CCGACGCCTA CTACTGTAGG


CCCGACTGGG ACGCGCTGAA GGCGACACGC TGA
GGGCTGACCC TGCGCGACTT CCGCTGTGCG ACT
```

Segment No. II

```
A TGATTGATTC AGCCAACAGA GCCGACGTCT
T ACTAACTAAG TCGGTTGTCT CGGCTGCAGA


TTCTCCGCAA GCCGGCACCC GTAGCGCCCG AACTGCAGCA
AAGAGGCGTT CGGCCGTGGG CATCGCGGGC TTGACGTCGT


CGAAGTCGAG CAGTTCTACT ATTGGGAGGC CAAGCTTCTC
GCTTCAGCTC GTCAAGATGA TAACCCTCCG GTTCGAAGAG


AACGATCGCC GCTTCGAGGA GTGGTTCGCG CTGCTCGCGG
TTGCTAGCGG CGAAGCTCCT CACCAAGCGC GACGAGCGCC


AAGACATTCA CTACTTCATG CCCATTCGCA CCACGCGGAT
TTCTGTAAGT GATGAAGTAC GGGTAAGCGT GGTGCGCCTA


CATGCGGGAC TCGCGCCTTG AATACTCAGG CTCCCGAGAG
GTACGCCCTG AGCGCGGAAC TTATGAGTCC GAGGGCTCTC


TACGCGCACT TCGATGACGA CGCCACGATG ATGAAGGGAC
ATGCGCGTGA AGCTACTGCT GCGGTGCTAC TACTTCCCTG


GCTTGCGCAA GATCACGTCC GACGTGAGCT GGTCCGAGAA
CGAACGCGTT CTAGTGCAGG CTGCACTCGA CCAGGCTCTT
```

6

```
CCCCGCATCG  CGGACCCGGC  ATCTCGTGAG  CAACGTGATG
GGGGCGTAGC  GCCTGGGCCG  TAGAGCACTC  GTTGCACTAC

ATCGTCGGCG  CAGAGGCAGA  AGGGGAGTAC  GAAATCTCAA
TAGCAGCCGC  GTCTCCGTCT  TCCCCTCATG  CTTTAGAGTT


GCGCCTTCAT  TGTGTACCGC  AATCGTCTGG  AGCGGCAGCT
CGCGGAAGTA  ACACATGGCG  TTAGCAGACC  TCGCCGTCGA


CGACATCTTT  GCCGGTGAGC  GTCGCGATAC  GTTGCGCCGT
GCTGTAGAAA  CGGCCACTCG  CAGCGCTATG  CAACGCGGCA


AACACGAGCG  AGGCCGGGTT  CGAGATCGTC  AATCGGACCA
TTGTGCTCGC  TCCGGCCCAA  GCTCTAGCAG  TTAGCCTGGT


TCCTGATCGA  CCAGAGCACC  ATCCTGGCCA  ATAACCTCAG
AGGACTAGCT  GGTCTCGTGG  TAGGACCGGT  TATTGGAGTC


TTTCTTCTTC  TAG
AAAGAAGAAG  ATC
```

Segment No. III

```
ATGGCTAC  CCATGTGGCG  ATCATCGGCA  ATGGCGTGGG
TACCGATG  GGTACACCGC  TAGTAGCCGT  TACCGCACCC


TGGCTTCACG  ACCGCGCAGG  CCCTACGTGC  CGAGGGCTTC
ACCGAAGTGC  TGGCGCGTCC  GGGATGCACG  GCTCCCGAAG


GAGGGGAGAA  TCTCGCTGAT  TGGGGACGAA  CCGCATCTCC
CTCCCCTCTT  AGAGCGACTA  ACCCCTGCTT  GGCGTAGAGG


CCTATGACCG  ACCATCCTTG  TCCAAGGCGG  TTCTCGACGG
GGATACTGGC  TGGTAGGAAC  AGGTTCCGCC  AAGAGCTGCC


CAGCCTTGAG  CGGCCGCCCA  TACTGGCCGA  GGCCGATTGG
GTCGGAACTC  GCCGGCGGGT  ATGACCGGCT  CCGGCTAACC
```

```
TACGGCGAGG CCCGCATCGA CATGCTGACC GGCCCGGAAG
ATGCCGCTCC GGGCGTAGCT GTACGACTGG CCGGGCCTTC


TCACTGCCCT TGATGTGCAG ACAAGGACGA TCAGTCTGGA
AGTGACGGGA ACTACACGTC TGTTCCTGCT AGTCAGACCT


TGATGGCACC ACGCTCTCTG CGGACGCCAT CGTCATCGCG
ACTACCGTGG TGCGAGAGAC GCCTGCGGTA GCAGTAGCGC


ACGGGCAGTC GAGCGCGGAC GATGGCGTTG CCCGGCAGCC
TGCCCGTCAG CTCGCGCCTG CTACCGCAAC GGGCCGTCGG


AACTGCCCGG CGTCGTAACG CTGCGCACCT ACGGTGACGT
TTGACGGGCC GCAGCATTGC GACGCGTGGA TGCCACTGCA


GCAGGTATTG CGCGATAGTT GGACTTCCGC GACGCGGCTG
CGTCCATAAC GCGCTATCAA CCTGAAGGCG CTGCGCCGAC


CTGATTGTGG GTGGCGGATT GATCGGCTGC GAGGTCGCGA
GACTAACACC CACCGCCTAA CTAGCCGACG CTCCAGCGCT


CGACGGCGCG CAAGCTCGGC CTGTCGGTCA CGATCCTGGA
GCTGCCGCGC GTTCGAGCCG GACAGCCAGT GCTAGGACCT


GGCAGGTGAT GAACTGCTGG TCCGAGTACT TGGGCGGCGT
CCGTCCACTA CTTGACGACC AGGCTCATGA ACCCGCCGCA


ATCGGTGCCT GGCTGCGCGG CCTGCTGACA GAACTTGGTG
TAGCCACGGA CCGACGCGCC GGACGACTGT CTTGAACCAC


TGCAGGTCGA GTTGGGAACG GGTGTCGTAG GTTTTTCTGG
ACGTCCAGCT CAACCCTTGC CCACAGCATC CAAAAAGACC


TGAGGGCCAG CTCGAACAAG TCATGGCCAG CGATGGGCGC
ACTCCCGGTC GAGCTTGTTC AGTACCGGTC GCTACCCGCG


AGCTTCGTAG CCGATAGCGC ACTCATTTGC GTCGGCGCGG
TCGAAGCATC GGCTATCGCG TGAGTAAACG CAGCCGCGCC
```

```
AGCCCGCGGA  TCAACTTGCG  CGTCAAGCGG  GCTTGGCATG
TCGGGCGCCT  AGTTGAACGC  GCAGTTCGCC  CGAACCGTAC


TGACCGCGGC  GTCATTGTCG  ATCACTGCGG  TGCGACGCTT
ACTGGCGCCG  CAGTAACAGC  TAGTGACGCC  ACGCTGCGAA


GCCAAAGGCT  ATTCGCCGTC  GGAGATGTGG  CCAGTTGGCG
CGGTTTCCGA  TAAGCGGCAG  CCTCTACACC  GGTCAACCGC


CTGCGGCCGG  CGGCCGGCGT  TCGCTCGAAA  CCTATATGAA
GACGCCGGCC  GCCGGCCGCA  AGCGAGCTTT  GGATATACTT


CGCGCAGCGC  CAAGCCGCCG  CGGTGGCTGC  GGCCATTCTG
GCGCGTCGCG  GTTCGGCGGC  GCCACCGACG  CCGGTAAGAC


GGGAAAAACG  TATCGGCACC  GCAACTGCCC  GTGTCCTGGA
CCCTTTTTGC  ATAGCCGTGG  CGTTGACGGG  CACAGGACCT


CGGAGATCGC  TGGGCATCGC  ATGCAGATGG  CGGGCGATAT
GCCTCTAGCG  ACCCGTAGCG  TACGTCTACC  GCCCGCTATA


CGAAGGACCT  GGTGATTTCG  TCTCGCGCGG  CATGCCCGGT
GCTTCCTGGA  CCACTAAAGC  AGAGCGCGCC  GTACGGGCCA


AGTGGCGCTG  CCCTGTTGTT  CCGCCTGCAG  GAGCGAAGGA
TCACCGCGAC  GGGACAACAA  GGCGGACGTC  CTCGCTTCCT


TTCAGGCGGT  CGTCGCGGTC  GATGCACCCC  GTGACTTCGC
AAGTCCGCCA  GCAGCGCCAG  CTACGTGGGG  CACTGAAGCG


GCTTGCAACC  CGATTGGTAG  AAGCCCGCGC  GGCAATCGAG
CGAACGTTGG  GCTAACCATC  TTCGGGCGCG  CCGTTAGCTC


CCAGCACGGC  TCGCAGATCT  TTCAAACAGT  ATGCGCGATT
GGTCGTGCCG  AGCGTCTAGA  AAGTTTGTCA  TACGCGCTAA


TTGTTCGTGC  GAATGAAGGA  GACCTAACGT  GA
AACAAGCACG  CTTACTTCCT  CTGGATTGCA  CT
```

The invention also relates to DNA sequences which are degenerate as a result of the genetic code to any of the foregoing DNA sequences and which code for a polypeptide having benzene oxygenase activity.

According to the second aspect of this invention, there is provided a prokaryotic cell which has been transformed with a recombinant DNA consisting of the DNA sequence as defined above and a vector DNA.

Further, according to the third aspect of this invention, there is provided a process for the production of 1,2-dihydroxy-cyclohexa-3,5-diene, which comprises treating an aromatic compound with the transformed prokaryotic cells as defined above.

The DNA sequence encoding a polypeptide having benzene oxygenase activity (benzene oxygenase gene) may be prepared by the following procedure:

Cosmid pGR300 prepared from an E. coli strain (SI-300, FERM BP-1358) which contains the benzene oxygenase gene and the benzene glycol dehydrogenase gene, was subjected to a known treatment such as the one described in "T. Maniatis, E.F. Fritsch, J. Sambrook, Molecular Cloning, A Laboratory Manual, Cold Spring Harbor Laboratory Press, 1982" to prepare a mini-plasmid containing the desired gene. The plasmid thus obtained was submitted to prepare transformed cells of a E. coli strain. Plasmid pGR115 was isolated from the transformed cells of an Escherichia coli strain, and was cut with several restriction enzymes, so that the restriction enzyme cleavage map as shown in Fig. 1 was prepared. DNA fragments obtained from the plasmid pGR115 by cleavage with several restriction enzymes as mentioned above was subjected to agarose gel electrophoresis, thereby obtaining a DNA fragment of about 4.2 Kb. This DNA fragment was combined with a vector plasmid and the combined product was introduced (transferred) into cells of E. coli strain, so that transformant cells were obtained. Plasmids thus obtained from transformant cells of the E. coli strain prepared as above were cut with several restriction enzymes and analyzed for identification, so that the E. coli JM109 strain which carries plasmid pGR1153 containing DNA fragments derived from plasmid pGR115 was selected. The segments of the base sequence which correspond to the desired gene were thus selected. The base sequences of the DNA segments thus obtained were determined according to M13 phage-dideoxy method. In the base sequence as determined, there are contained at least three DNA segments in combination coding for a polypeptide having benzene oxygenase activity, namely DNA segments designated as I, II and III.

The benzene oxygenase gene thus isolated may be introduced, in a known manner, into cells of microorganisms, particularly of prokaryotic microorganisms which lack the benzene oxygenase activity. Various prokaryotic microorganisms, preferably Escherichia coli may be used in this invention.

1,2-Dihydroxy-cyclohexa-3,5-diene can be prepared effectively in a high yield by allowing an aromatic compound to react, in contact with transformed cells as described above of a prokaryotic microorganism to effect the conversion of said aromatic compound into the desired compound, namely 1,2-dihydroxy-cyclohexa-3,5-diene.Preferred examples of aromatic compounds are benzene, toluene, biphenyl or naphthalene. The reaction is carried out in the presence of assimilable carbon sources such as ethanol, acetic acid, glucose and lactic acid, whereby the aromatic compound is converted into 1,2-dihydroxy-cyclohexa-3,5-diene. The reaction may be conducted at a temperature from 10 to 45°C, preferably from 25 to 35°C. After the completion of the reaction, the reaction solution is concentrated, and the resulting concentrate is extracted with an appropriate organic solvent such as diethyl ether or methylene chloride. From the extract, after having been concentrated if necessary, there is recovered 1,2-dihydroxy-cyclohexa-3,5-diene in a high yield.

1,2-Dihydroxy-cyclohexa-3,5-diene finds various practical uses such as in the production of engineering plastics.

This invention will be illustrated in more detail with respect to the following examples. It should be understood that these examples should not be construed as limiting the scope of this invention.

## Example 1

Cosmid pGR300 derived from the cells of Escherichia coli SI-300 (FERM BP-1358), which contains the benzene oxygenase gene and the benzene glycol dehydrogenase gene was cut with restriction enzyme EcoRI to obtain a cleaved DNA fragment. The DNA fragment thus obtained was ligated with plasmid pUC8 cut with EcoRI (See: Gene, 19, 259 (1982)) and the ligated product was introduced into the cells of the Escherichia coli JM83 strain.

The cleavage of cosmid pGR300 with the restriction enzyme EcoRI, the ligation of the DNA fragment with the cleaved plasmid pUC8 and the transformation of the cells of Escherichia coli was performed in a known manner according, for example, to "T. Maniatis, E.F. Fritsch, J. Sambrook, Molecular Cloning, A Laboratory Manual, Cold Spring Harbor Laboratory Press, 1982".

10

The transformant cells of Escherichia coli were subjected to a known isolation procedure for plasmids. The isolated plasmids were cut with restriction enzyme and analyzed in a conventional manner, so that a restriction enzyme cleavage map was prepared. Through cleavage with restriction enzyme and treatment with exonuclease Bal-31 in a conventional manner which was carried out based on the restriction enzyme cleavage map mentioned above, a deletion product, i.e. plasmid pGR115, was prepared.

The plasmid pGR115 was cleaved with restriction enzyme PvuII to obtain a DNA fragment which was then submitted to agarose gel electrophoresis, whereby a DNA fragment of about 4.2 Kb was selectively isolated from the gel. The DNA fragment thus obtained was ligated to plasmid pUC18 (a product of Takara Shuzo Co., Ltd.) cleaved with restriction enzyme SmaI, and the ligated DNA fragments were introduced (transferred) into the cells of the Escherichia coli JM109 strain, to obtain transformant cells which grow in a LB medium containing Ampicillin 100 μg/ml. The cleavage of plasmid pGR115 as well as of plasmid pUC18 with the corresponding restriction enzymes, agarose-gel electrophoresis of DNA fragments, isolation of DNA fragments from gel, ligation of DNA fragments with plasmid pUC18 and transformation of the cells of Escherichia coli were carried out in a conventional manner as described, for example in "T. Maniatis, E.F. Fritsch, J. Sambrook, Molecular Cloning, A Laboratory Manual, Cold Spring Harbor Laboratory Press, 1982 ".

From the transformant cells of Escherichia coli were isolated plasmids (7.0 Kb) which were then cut and analyzed in a conventional manner to identify the base sequence. As a result, Escherichia coli JM109 (FERM BP-1353) has been selected, which bears plasmid pGR1153 containing a DNA fragment of 4.2 Kb derived from plasmid pGR115.

It was found that the DNA fragment of 4.2 Kb thus obtained contains the DNA (nucleotide) segments Nos. I, II, III and a part of the DNA (nucleotide) segment No. IV. These four DNA (nucleotide) segments Nos. I, II, III and IV have been described as encoding respectively four particular proteins (See: Japanese Patent Application No. 68700/1986). The restriction enzyme cleavage map of the DNA segments contained in plasmids pGR115 and pGR1153 and isolated from the cells of microorganisms having a benzene-oxidizing ability, such as Escherichia coli , as shown in Fig. 1.

Example 2

The cells of the Escherichia coli JM109 strain which contain plasmids pGR115, pGR1153 and pUC18 were inoculated in a LB medium (100 ml) containing Ampicillin 100 μg/ml and cultured at 30°C for 16 hours. After the cultivation, the culture liquid was centrifuged to recover the cells which were then washed with phosphate buffer (pH 7.0) (50 mM) and further suspended in the phosphate buffer (50 ml). The suspension was placed in a 500 ml flask (Sakaguchi), admixed with benzene (100 μl) and ethanol (100 μl), and the resultant mixture was stirred at 30°C to effect the conversion of benzene. After a reaction time of 7 hours, the content of 1,2-dihydroxy-cyclohexa-3,5-diene in the reaction solution was determined by means of high-speed liquid chromatography (a product of Nihon Bunko Co., Ltd.), on the column of Finepac C-18, and detected by the absorption at 265nm.

The result is shown in the Table 1 below;

### Table 1

| Strain of Microorganism | Yield (g/l) |
|---|---|
| Escherichia coli JM109 (pUC18) | 0 |
| " JM109 (pGR115) | 0 |
| " JM109 (pGR1153) | 2.6 |

Example 3

The cells of Escherichia coli (FERM BP-1353) containing plasmids pGR1153 were treated in the same manner as in the Example 2, to obtain a reaction solution.

11

The reaction solution (200 ml) thus obtained was concentrated to a volume of about 30 ml by a rotary evaporator. The concentrate was extracted successively with portions (each 50 ml) of diethyl ether. The extracts were combined and dried and evaporated to remove the diethyl ether. The solid residue was recrystallized from a mixture of methylene chloride and pentane, to obtain crystalls which were submitted to the following test for identification of the product.

(1) Analysis by NMR (proton) (270 MHz); NMR (deuterium) shows signals at 4.2 ppm (C-H, 2H), 4.6 ppm (O-H, 2H) and 5.9 ppm (C = C, 4H), which identifies the sample as cis-1,2-dihydroxy-cyclohexa-3,5-diene.

(2) Analysis by GC-MS (Gas Chromatography-Mass Spectrometry) GC-MS conducted on a column SE-30 (2 m), at 100°C (temperature on injection: 250°C), identified the sample as corresponding to cis-1,2-dihydroxy-cyclohexa-3,5-diene, in view of its molecular weight of 112.

**Claims**

1. A DNA sequence containing the following DNA segments Nos. I, II and III,in combination, encoding a polypeptide having benzene oxygenase activity, said segments having the following formulae:

```
Segment No. I


ATGA  ATCAGACCGA  CACATCACCT  ATCAGGCTGC
TACT  TAGTCTGGCT  GTGTAGTGGA  TAGTCCGACG


GCAGGAGCTG  GAACACCAGC  GAGATAGAAG  CGCTCTTTGA
CGTCCTCGAC  CTTGTGGTCG  CTCTATCTTC  GCGAGAAACT


CGAGCATGCC  GGACGTATCG  ATCCGCGCAT  TTATACCGAT
GCTCGTACGG  CCTGCATAGC  TAGGCGCGTA  AATATGGCTA


GAGGATCTGT  ACCAACTCGA  ACTGGAGCGT  GTCTTCGCCC
CTCCTAGACA  TGGTTGAGCT  TGACCTCGCA  CAGAAGCGGG


GGTCCTGGCT  GCTGTTGGGG  CATGAAACCC  AGATTCGCAA
CCAGGACCGA  CGACAACCCC  GTACTTTGGG  TCTAAGCGTT


GCCGGGCGAT  TACATCACGA  CCTACATGGG  TGAAGACCCT
CGGCCCGCTA  ATGTAGTGCT  GGATGTACCC  ACTTCTGGGA


GTCGTGGTCG  TCCGGCAGAA  AGACGCCAGC  ATTGCCGTGT
CAGCACCAGC  AGGCCGTCTT  TCTGCGGTCG  TAACGGCACA


TCCTGAACCA  GTGCCGCCAC  CGTGGCATGC  GCATCTGCCG
AGGACTTGGT  CACGGCGGTG  GCACCGTACG  CGTAGACGGC


CGCGGATGCC  GGAAACGCGA  AGGCGTTCAC  TTGCAGCTAC
GCGCCTACGG  CCTTTGCGCT  TCCGCAAGTG  AACGTCGATG
```

```
CACGGGTGGG CTTACGACAC CGCCGGCAAT CTTGTCAATG
GTGCCCACCC GAATGCTGTG GCGGCCGTTA GAACAGTTAC


TGCCTTACGA GGCCGAATCC TTCGCGTGCC TGAACAAGAA
ACGGAATGCT CCGGCTTAGG AAGCGCACGG ACTTGTTCTT


GGAATGGAGC CCGCTGAAGG CCCGGGTAGA AACCTACAAG
CCTTACCTCG GGCGACTTCC GGGCCCATCT TTGGATGTTC


GGCCTGATTT TCGCCAACTG GGATGAGAAC GCTGTAGACC
CCGGACTAAA AGCGGTTGAC CCTACTCTTG CGACATCTGG


TCGACACGTA TCTGGGCGAG GCGAAGTTCT ACATGGACCA
AGCTGTGCAT AGACCCGCTC CGCTTCAAGA TGTACCTGGT


CATGCTCGAC CGCACCGAGG CCGGCACCGA AGCGATCCCG
GTACGAGCTG GCGTGGCTCC GGCCGTGGCT TCGCTAGGGC


GGCGTGCAGA AGTGGGTCAT TCCCTGTAAC TGGAAATTCG
CCGCACGTCT TCACCCAGTA AGGGACATTG ACCTTTAAGC


CCGCAGAGCA GTTTTGCAGC GACATGTACC ATGCCGGGAC
GGCGTCTCGT CAAAACGTCG CTGTACATGG TACGGCCCTG


GACCTCGCAT CTGTCTGGCA TCCTGGCAGG CCTGCCAGAA
CTGGAGCGTA GACAGACCGT AGGACCGTCC GGACGGTCTT


GACCTTGAAA TGGCCGACCT TGCTCCGCCG ACAGTTGGCA
CTGGAACTTT ACCGGCTGGA ACGAGGCGGC TGTCAACCGT


AGCAGTACCG TGCGTCATGG GGCGGACATG GAAGTGGCTT
TCGTCATGGC ACGCAGTACC CCGCCTGTAC CTTCACCGAA


CTATGTCGGC GACCCCAATC TGATGCTTGC CATCATGGGG
GATACAGCCG CTGGGGTTAG ACTACGAACG GTAGTACCCC
```

```
CCAAAGGTCA  CCAGCTACTG  GACCGAAGGC  CCCGCGTCGG
GGTTTCCAGT  GGTCGATGAC  CTGGCTTCCG  GGGCGCAGCC


AAAAGGCGGC  CGAACGTCTG  GGTAGCGTGG  AGCGCGGCTC
TTTTCCGCCG  GCTTGCAGAC  CCATCGCACC  TCGCGCCGAG


GAAACTCATG  GTCGAGCACA  TGACCGTCTT  CCCCACGTGT
CTTTGAGTAC  CAGCTCGTGT  ACTGGCAGAA  GGGGTGCACA


TCCTTCCTCC  CAGGTATCAA  TACGGTCCGG  ACATTGGCAT
AGGAAGGAGG  GTCCATAGTT  ATGCCAGGCC  TGTAACCGTA


CCGCGCGGGC  CGAACGAGGT  GAGGTATGGG  CGTTTACGGT
GGCGCGCCCG  GCTTGCTCCA  CTCCATACCC  GCAAATGCCA


GGTCGATGCT  GATGCTCCTG  ACGATATCAA  GGAAGAGTTC
CCAGCTACGA  CTACGAGGAC  TGCTATAGTT  CCTTCTCAAG


CGCGCCAGAC  TGCGCACCTT  CTCTCCGGTG  GCGTGTTCGA
GCGCGGTCTG  ACGCGTGGAA  GAGAGGCCAC  CGCACAAGCT


GCAGGACGAC  GGGGAGAACT  GGGTCGAGAT  CCAGCACATC
CGTCCTGCTG  CCCCTCTTGA  CCCAGCTCTA  GGTCGTGTAG


CTGCGAGGCC  ACAAGCCGGA  GCCGCCCTTT  CAATGCCGAG
GACGCTCCGG  TGTTCGGCCT  CGGCGGGAAA  GTTACGGCTC


ATGAGCATGG  ACCAGACCGT  CGACAACGAC  CCGGTTTACC
TACTCGTACC  TGGTCTGGCA  GCTGTTGCTG  GGCCAAATGG


CCGGGCGGAT  CAGCAACAAC  GTCTACAGCG  AGGAAGCTGC
GGCCCGCCTA  GTCGTTGTTG  CAGATGTCGC  TCCTTCGACG


CCGCGGGCTC  TATGCCCATT  GGCTGCGGAT  GATGACATCC
GGCGCCCGAG  ATACGGGTAA  CCGACGCCTA  CTACTGTAGG
```

```
CCCGACTGGG ACGCGCTGAA GGCGACACGC TGA
GGGCTGACCC TGCGCGACTT CCGCTGTGCG ACT
```

Segment No. II

```
A TGATTGATTC AGCCAACAGA GCCGACGTCT
T ACTAACTAAG TCGGTTGTCT CGGCTGCAGA
```

```
TTCTCCGCAA GCCGGCACCC GTAGCGCCCG AACTGCAGCA
AAGAGGCGTT CGGCCGTGGG CATCGCGGGC TTGACGTCGT
```

```
CGAAGTCGAG CAGTTCTACT ATTGGGAGGC CAAGCTTCTC
GCTTCAGCTC GTCAAGATGA TAACCCTCCG GTTCGAAGAG
```

```
AACGATCGCC GCTTCGAGGA GTGGTTCGCG CTGCTCGCGG
TTGCTAGCGG CGAAGCTCCT CACCAAGCGC GACGAGCGCC
```

```
AAGACATTCA CTACTTCATG CCCATTCGCA CCACGCGGAT
TTCTGTAAGT GATGAAGTAC GGGTAAGCGT GGTGCGCCTA
```

```
CATGCGGGAC TCGCGCCTTG AATACTCAGG CTCCCGAGAG
GTACGCCCTG AGCGCGGAAC TTATGAGTCC GAGGGCTCTC
```

```
TACGCGCACT TCGATGACGA CGCCACGATG ATGAAGGGAC
ATGCGCGTGA AGCTACTGCT GCGGTGCTAC TACTTCCCTG
```

```
GCTTGCGCAA GATCACGTCC GACGTGAGCT GGTCCGAGAA
CGAACGCGTT CTAGTGCAGG CTGCACTCGA CCAGGCTCTT
```

```
CCCCGCATCG CGGACCCGGC ATCTCGTGAG CAACGTGATG
GGGGCGTAGC GCCTGGGCCG TAGAGCACTC GTTGCACTAC
```

```
ATCGTCGGCG CAGAGGCAGA AGGGGAGTAC GAAATCTCAA
TAGCAGCCGC GTCTCCGTCT TCCCCTCATG CTTTAGAGTT
```

15

```
GCGCCTTCAT  TGTGTACCGC  AATCGTCTGG  AGCGGCAGCT
CGCGGAAGTA  ACACATGGCG  TTAGCAGACC  TCGCCGTCGA

CGACATCTTT  GCCGGTGAGC  GTCGCGATAC  GTTGCGCCGT
GCTGTAGAAA  CGGCCACTCG  CAGCGCTATG  CAACGCGGCA

AACACGAGCG  AGGCCGGGTT  CGAGATCGTC  AATCGGACCA
TTGTGCTCGC  TCCGGCCCAA  GCTCTAGCAG  TTAGCCTGGT

TCCTGATCGA  CCAGAGCACC  ATCCTGGCCA  ATAACCTCAG
AGGACTAGCT  GGTCTCGTGG  TAGGACCGGT  TATTGGAGTC

TTTCTTCTTC  TAG
AAAGAAGAAG  ATC
```

Segment No. III

```
ATGGCTAC  CCATGTGGCG  ATCATCGGCA  ATGGCGTGGG
TACCGATG  GGTACACCGC  TAGTAGCCGT  TACCGCACCC

TGGCTTCACG  ACCGCGCAGG  CCCTACGTGC  CGAGGGCTTC
ACCGAAGTGC  TGGCGCGTCC  GGGATGCACG  GCTCCCGAAG

GAGGGGAGAA  TCTCGCTGAT  TGGGGACGAA  CCGCATCTCC
CTCCCCTCTT  AGAGCGACTA  ACCCTGCTT  GGCGTAGAGG

CCTATGACCG  ACCATCCTTG  TCCAAGGCGG  TTCTCGACGG
GGATACTGGC  TGGTAGGAAC  AGGTTCCGCC  AAGAGCTGCC

CAGCCTTGAG  CGGCCGCCCA  TACTGGCCGA  GGCCGATTGG
GTCGGAACTC  GCCGGCGGGT  ATGACCGGCT  CCGGCTAACC

TACGGCGAGG  CCCGCATCGA  CATGCTGACC  GGCCCGGAAG
ATGCCGCTCC  GGGCGTAGCT  GTACGACTGG  CCGGGCCTTC

TCACTGCCCT  TGATGTGCAG  ACAAGGACGA  TCAGTCTGGA
AGTGACGGGA  ACTACACGTC  TGTTCCTGCT  AGTCAGACCT
```

```
TGATGGCACC ACGCTCTCTG CGGACGCCAT CGTCATCGCG
ACTACCGTGG TGCGAGAGAC GCCTGCGGTA GCAGTAGCGC


ACGGGCAGTC GAGCGCGGAC GATGGCGTTG CCCGGCAGCC
TGCCCGTCAG CTCGCGCCTG CTACCGCAAC GGGCCGTCGG


AACTGCCCGG CGTCGTAACG CTGCGCACCT ACGGTGACGT
TTGACGGGCC GCAGCATTGC GACGCGTGGA TGCCACTGCA


GCAGGTATTG CGCGATAGTT GGACTTCCGC GACGCGGCTG
CGTCCATAAC GCGCTATCAA CCTGAAGGCG CTGCGCCGAC


CTGATTGTGG GTGGCGGATT GATCGGCTGC GAGGTCGCGA
GACTAACACC CACCGCCTAA CTAGCCGACG CTCCAGCGCT


CGACGGCGCG CAAGCTCGGC CTGTCGGTCA CGATCCTGGA
GCTGCCGCGC GTTCGAGCCG GACAGCCAGT GCTAGGACCT


GGCAGGTGAT GAACTGCTGG TCCGAGTACT TGGGCGGCGT
CCGTCCACTA CTTGACGACC AGGCTCATGA ACCCGCCGCA


ATCGGTGCCT GGCTGCGCGG CCTGCTGACA GAACTTGGTG
TAGCCACGGA CCGACGCGCC GGACGACTGT CTTGAACCAC


TGCAGGTCGA GTTGGGAACG GGTGTCGTAG GTTTTTCTGG
ACGTCCAGCT CAACCCTTGC CCACAGCATC CAAAAAGACC


TGAGGGCCAG CTCGAACAAG TCATGGCCAG CGATGGGCGC
ACTCCCGGTC GAGCTTGTTC AGTACCGGTC GCTACCCGCG


AGCTTCGTAG CCGATAGCGC ACTCATTTGC GTCGGCGCGG
TCGAAGCATC GGCTATCGCG TGAGTAAACG CAGCCGCGCC


AGCCCGCGGA TCAACTTGCG CGTCAAGCGG GCTTGGCATG
TCGGGCGCCT AGTTGAACGC GCAGTTCGCC CGAACCGTAC


TGACCGCGGC GTCATTGTCG ATCACTGCGG TGCGACGCTT
ACTGGCGCCG CAGTAACAGC TAGTGACGCC ACGCTGCGAA
```

```
GCCAAAGGCT  ATTCGCCGTC  GGAGATGTGG  CCAGTTGGCG
CGGTTTCCGA  TAAGCGGCAG  CCTCTACACC  GGTCAACCGC


CTGCGGCCGG  CGGCCGGCGT  TCGCTCGAAA  CCTATATGAA
GACGCCGGCC  GCCGGCCGCA  AGCGAGCTTT  GGATATACTT


CGCGCAGCGC  CAAGCCGCCG  CGGTGGCTGC  GGCCATTCTG
GCGCGTCGCG  GTTCGGCGGC  GCCACCGACG  CCGGTAAGAC


GGGAAAAACG  TATCGGCACC  GCAACTGCCC  GTGTCCTGGA
CCCTTTTTGC  ATAGCCGTGG  CGTTGACGGG  CACAGGACCT


CGGAGATCGC  TGGGCATCGC  ATGCAGATGG  CGGGCGATAT
GCCTCTAGCG  ACCCGTAGCG  TACGTCTACC  GCCCGCTATA


CGAAGGACCT  GGTGATTTCG  TCTCGCGCGG  CATGCCCGGT
GCTTCCTGGA  CCACTAAAGC  AGAGCGCGCC  GTACGGGCCA


AGTGGCGCTG  CCCTGTTGTT  CCGCCTGCAG  GAGCGAAGGA
TCACCGCGAC  GGGACAACAA  GGCGGACGTC  CTCGCTTCCT


TTCAGGCGGT  CGTCGCGGTC  GATGCACCCC  GTGACTTCGC
AAGTCCGCCA  GCAGCGCCAG  CTACGTGGGG  CACTGAAGCG


GCTTGCAACC  CGATTGGTAG  AAGCCCGCGC  GGCAATCGAG
CGAACGTTGG  GCTAACCATC  TTCGGGCGCG  CCGTTAGCTC


CCAGCACGGC  TCGCAGATCT  TTCAAACAGT  ATGCGCGATT
GGTCGTGCCG  AGCGTCTAGA  AAGTTTGTCA  TACGCGCTAA


TTGTTCGTGC  GAATGAAGGA  GACCTAACGT  GA
AACAAGCACG  CTTACTTCCT  CTGGATTGCA  CT, and
```

DNA sequences which, based on the degeneration of the genetic code, correspond to any of the foregoing DNA sequences and which code a polypeptide having benzene oxygenase activity.

2.   A prokaryotic cell transformed with a recombinant DNA which contains the following DNA segments Nos. I, II and III localized therein, in combination, and which encodes a polypeptide having benzene oxygenase activity, and a vector DNA; said segments having the formulae as defined in claim 1.

3.   A cell according to claim 2, wherein the prokaryote is Escherichia coli.

18

4. A process for the production of 1,2-dihydroxy-cyclohexa-3,5-diene, which comprises treating an aromatic compound with prokaryotic cells transformed with a recombinant DNA which contains the DNA segments Nos. I, II and III as defined in claim 1.

5. A process according to claim 4, wherein the prokaryote is Escherichia coli.

**Revendications**

1. Séquence d'ADN contenant les segments d'ADN no I, II et III en combinaison, codant pour un polypeptide qui possède une activité benzène oxygénase, lesdits segments correspondant aux formules suivantes:

```
Segment No. I


ATGA  ATCAGACCGA  CACATCACCT  ATCAGGCTGC
TACT  TAGTCTGGCT  GTGTAGTGGA  TAGTCCGACG


GCAGGAGCTG  GAACACCAGC  GAGATAGAAG  CGCTCTTTGA
CGTCCTCGAC  CTTGTGGTCG  CTCTATCTTC  GCGAGAAACT


CGAGCATGCC  GGACGTATCG  ATCCGCGCAT  TTATACCGAT
GCTCGTACGG  CCTGCATAGC  TAGGCGCGTA  AATATGGCTA


GAGGATCTGT  ACCAACTCGA  ACTGGAGCGT  GTCTTCGCCC
CTCCTAGACA  TGGTTGAGCT  TGACCTCGCA  CAGAAGCGGG


GGTCCTGGCT  GCTGTTGGGG  CATGAAACCC  AGATTCGCAA
CCAGGACCGA  CGACAACCCC  GTACTTTGGG  TCTAAGCGTT


GCCGGGCGAT  TACATCACGA  CCTACATGGG  TGAAGACCCT
CGGCCCGCTA  ATGTAGTGCT  GGATGTACCC  ACTTCTGGGA


GTCGTGGTCG  TCCGGCAGAA  AGACGCCAGC  ATTGCCGTGT
CAGCACCAGC  AGGCCGTCTT  TCTGCGGTCG  TAACGGCACA


TCCTGAACCA  GTGCCGCCAC  CGTGGCATGC  GCATCTGCCG
AGGACTTGGT  CACGGCGGTG  GCACCGTACG  CGTAGACGGC


CGCGGATGCC  GGAAACGCGA  AGGCGTTCAC  TTGCAGCTAC
GCGCCTACGG  CCTTTGCGCT  TCCGCAAGTG  AACGTCGATG
```

```
CACGGGTGGG CTTACGACAC CGCCGGCAAT CTTGTCAATG
GTGCCCACCC GAATGCTGTG GCGGCCGTTA GAACAGTTAC


TGCCTTACGA GGCCGAATCC TTCGCGTGCC TGAACAAGAA
ACGGAATGCT CCGGCTTAGG AAGCGCACGG ACTTGTTCTT


GGAATGGAGC CCGCTGAAGG CCCGGGTAGA AACCTACAAG
CCTTACCTCG GGCGACTTCC GGGCCCATCT TTGGATGTTC


GGCCTGATTT TCGCCAACTG GGATGAGAAC GCTGTAGACC
CCGGACTAAA AGCGGTTGAC CCTACTCTTG CGACATCTGG


TCGACACGTA TCTGGGCGAG GCGAAGTTCT ACATGGACCA
AGCTGTGCAT AGACCCGCTC CGCTTCAAGA TGTACCTGGT


CATGCTCGAC CGCACCGAGG CCGGCACCGA AGCGATCCCG
GTACGAGCTG GCGTGGCTCC GGCCGTGGCT TCGCTAGGGC


GGCGTGCAGA AGTGGGTCAT TCCCTGTAAC TGGAAATTCG
CCGCACGTCT TCACCCAGTA AGGGACATTG ACCTTTAAGC


CCGCAGAGCA GTTTTGCAGC GACATGTACC ATGCCGGGAC
GGCGTCTCGT CAAAACGTCG CTGTACATGG TACGGCCCTG


GACCTCGCAT CTGTCTGGCA TCCTGGCAGG CCTGCCAGAA
CTGGAGCGTA GACAGACCGT AGGACCGTCC GGACGGTCTT


GACCTTGAAA TGGCCGACCT TGCTCCGCCG ACAGTTGGCA
CTGGAACTTT ACCGGCTGGA ACGAGGCGGC TGTCAACCGT


AGCAGTACCG TGCGTCATGG GGCGGACATG GAAGTGGCTT
TCGTCATGGC ACGCAGTACC CCGCCTGTAC CTTCACCGAA


CTATGTCGGC GACCCCAATC TGATGCTTGC CATCATGGGG
GATACAGCCG CTGGGGTTAG ACTACGAACG GTAGTACCCC
```

20

```
CCAAAGGTCA CCAGCTACTG GACCGAAGGC CCCGCGTCGG
GGTTTCCAGT GGTCGATGAC CTGGCTTCCG GGGCGCAGCC


AAAAGGCGGC CGAACGTCTG GGTAGCGTGG AGCGCGGCTC
TTTTCCGCCG GCTTGCAGAC CCATCGCACC TCGCGCCGAG


GAAACTCATG GTCGAGCACA TGACCGTCTT CCCCACGTGT
CTTTGAGTAC CAGCTCGTGT ACTGGCAGAA GGGGTGCACA


TCCTTCCTCC CAGGTATCAA TACGGTCCGG ACATTGGCAT
AGGAAGGAGG GTCCATAGTT ATGCCAGGCC TGTAACCGTA


CCGCGCGGGC CGAACGAGGT GAGGTATGGG CGTTTACGGT
GGCGCGCCCG GCTTGCTCCA CTCCATACCC GCAAATGCCA


GGTCGATGCT GATGCTCCTG ACGATATCAA GGAAGAGTTC
CCAGCTACGA CTACGAGGAC TGCTATAGTT CCTTCTCAAG


CGCGCCAGAC TGCGCACCTT CTCTCCGGTG GCGTGTTCGA
GCGCGGTCTG ACGCGTGGAA GAGAGGCCAC CGCACAAGCT


GCAGGACGAC GGGGAGAACT GGGTCGAGAT CCAGCACATC
CGTCCTGCTG CCCCTCTTGA CCCAGCTCTA GGTCGTGTAG


CTGCGAGGCC ACAAGCCGGA GCCGCCCTTT CAATGCCGAG
GACGCTCCGG TGTTCGGCCT CGGCGGGAAA GTTACGGCTC


ATGAGCATGG ACCAGACCGT CGACAACGAC CCGGTTTACC
TACTCGTACC TGGTCTGGCA GCTGTTGCTG GGCCAAATGG


CCGGGCGGAT CAGCAACAAC GTCTACAGCG AGGAAGCTGC
GGCCCGCCTA GTCGTTGTTG CAGATGTCGC TCCTTCGACG


CCGCGGGCTC TATGCCCATT GGCTGCGGAT GATGACATCC
GGCGCCCGAG ATACGGGTAA CCGACGCCTA CTACTGTAGG
```

```
CCCGACTGGG ACGCGCTGAA GGCGACACGC TGA
GGGCTGACCC TGCGCGACTT CCGCTGTGCG ACT
```

Segment No. II

```
A TGATTGATTC AGCCAACAGA GCCGACGTCT
T ACTAACTAAG TCGGTTGTCT CGGCTGCAGA
```

```
TTCTCCGCAA GCCGGCACCC GTAGCGCCCG AACTGCAGCA
AAGAGGCGTT CGGCCGTGGG CATCGCGGGC TTGACGTCGT
```

```
CGAAGTCGAG CAGTTCTACT ATTGGGAGGC CAAGCTTCTC
GCTTCAGCTC GTCAAGATGA TAACCCTCCG GTTCGAAGAG
```

```
AACGATCGCC GCTTCGAGGA GTGGTTCGCG CTGCTCGCGG
TTGCTAGCGG CGAAGCTCCT CACCAAGCGC GACGAGCGCC
```

```
AAGACATTCA CTACTTCATG CCCATTCGCA CCACGCGGAT
TTCTGTAAGT GATGAAGTAC GGGTAAGCGT GGTGCGCCTA
```

```
CATGCGGGAC TCGCGCCTTG AATACTCAGG CTCCCGAGAG
GTACGCCCTG AGCGCGGAAC TTATGAGTCC GAGGGCTCTC
```

```
TACGCGCACT TCGATGACGA CGCCACGATG ATGAAGGGAC
ATGCGCGTGA AGCTACTGCT GCGGTGCTAC TACTTCCCTG
```

```
GCTTGCGCAA GATCACGTCC GACGTGAGCT GGTCCGAGAA
CGAACGCGTT CTAGTGCAGG CTGCACTCGA CCAGGCTCTT
```

```
CCCCGCATCG CGGACCCGGC ATCTCGTGAG CAACGTGATG
GGGGCGTAGC GCCTGGGCCG TAGAGCACTC GTTGCACTAC
```

```
ATCGTCGGCG CAGAGGCAGA AGGGGAGTAC GAAATCTCAA
TAGCAGCCGC GTCTCCGTCT TCCCCTCATG CTTTAGAGTT
```

```
GCGCCTTCAT TGTGTACCGC AATCGTCTGG AGCGGCAGCT
CGCGGAAGTA ACACATGGCG TTAGCAGACC TCGCCGTCGA


CGACATCTTT GCCGGTGAGC GTCGCGATAC GTTGCGCCGT
GCTGTAGAAA CGGCCACTCG CAGCGCTATG CAACGCGGCA


AACACGAGCG AGGCCGGGTT CGAGATCGTC AATCGGACCA
TTGTGCTCGC TCCGGCCCAA GCTCTAGCAG TTAGCCTGGT


TCCTGATCGA CCAGAGCACC ATCCTGGCCA ATAACCTCAG
AGGACTAGCT GGTCTCGTGG TAGGACCGGT TATTGGAGTC


TTTCTTCTTC TAG
AAAGAAGAAG ATC
```

Segment No. III

```
ATGGCTAC CCATGTGGCG ATCATCGGCA ATGGCGTGGG
TACCGATG GGTACACCGC TAGTAGCCGT TACCGCACCC


TGGCTTCACG ACCGCGCAGG CCCTACGTGC CGAGGGCTTC
ACCGAAGTGC TGGCGCGTCC GGGATGCACG GCTCCCGAAG


GAGGGGAGAA TCTCGCTGAT TGGGGACGAA CCGCATCTCC
CTCCCCTCTT AGAGCGACTA ACCCCTGCTT GGCGTAGAGG


CCTATGACCG ACCATCCTTG TCCAAGGCGG TTCTCGACGG
GGATACTGGC TGGTAGGAAC AGGTTCCGCC AAGAGCTGCC


CAGCCTTGAG CGGCCGCCCA TACTGGCCGA GGCCGATTGG
GTCGGAACTC GCCGGCGGGT ATGACCGGCT CCGGCTAACC


TACGGCGAGG CCCGCATCGA CATGCTGACC GGCCCGGAAG
ATGCCGCTCC GGGCGTAGCT GTACGACTGG CCGGGCCTTC


TCACTGCCCT TGATGTGCAG ACAAGGACGA TCAGTCTGGA
AGTGACGGGA ACTACACGTC TGTTCCTGCT AGTCAGACCT
```

```
TGATGGCACC  ACGCTCTCTG  CGGACGCCAT  CGTCATCGCG
ACTACCGTGG  TGCGAGAGAC  GCCTGCGGTA  GCAGTAGCGC


ACGGGCAGTC  GAGCGCGGAC  GATGGCGTTG  CCCGGCAGCC
TGCCCGTCAG  CTCGCGCCTG  CTACCGCAAC  GGGCCGTCGG


AACTGCCCGG  CGTCGTAACG  CTGCGCACCT  ACGGTGACGT
TTGACGGGCC  GCAGCATTGC  GACGCGTGGA  TGCCACTGCA


GCAGGTATTG  CGCGATAGTT  GGACTTCCGC  GACGCGGCTG
CGTCCATAAC  GCGCTATCAA  CCTGAAGGCG  CTGCGCCGAC


CTGATTGTGG  GTGGCGGATT  GATCGGCTGC  GAGGTCGCGA
GACTAACACC  CACCGCCTAA  CTAGCCGACG  CTCCAGCGCT


CGACGGCGCG  CAAGCTCGGC  CTGTCGGTCA  CGATCCTGGA
GCTGCCGCGC  GTTCGAGCCG  GACAGCCAGT  GCTAGGACCT


GGCAGGTGAT  GAACTGCTGG  TCCGAGTACT  TGGGCGGCGT
CCGTCCACTA  CTTGACGACC  AGGCTCATGA  ACCCGCCGCA


ATCGGTGCCT  GGCTGCGCGG  CCTGCTGACA  GAACTTGGTG
TAGCCACGGA  CCGACGCGCC  GGACGACTGT  CTTGAACCAC


TGCAGGTCGA  GTTGGGAACG  GGTGTCGTAG  GTTTTTCTGG
ACGTCCAGCT  CAACCCTTGC  CCACAGCATC  CAAAAAGACC


TGAGGGCCAG  CTCGAACAAG  TCATGGCCAG  CGATGGGCGC
ACTCCCGGTC  GAGCTTGTTC  AGTACCGGTC  GCTACCCGCG


AGCTTCGTAG  CCGATAGCGC  ACTCATTTGC  GTCGGCGCGG
TCGAAGCATC  GGCTATCGCG  TGAGTAAACG  CAGCCGCGCC


AGCCCGCGGA  TCAACTTGCG  CGTCAAGCGG  GCTTGGCATG
TCGGGCGCCT  AGTTGAACGC  GCAGTTCGCC  CGAACCGTAC


TGACCGCGGC  GTCATTGTCG  ATCACTGCGG  TGCGACGCTT
ACTGGCGCCG  CAGTAACAGC  TAGTGACGCC  ACGCTGCGAA
```

```
GCCAAAGGCT ATTCGCCGTC GGAGATGTGG CCAGTTGGCG
CGGTTTCCGA TAAGCGGCAG CCTCTACACC GGTCAACCGC

CTGCGGCCGG CGGCCGGCGT TCGCTCGAAA CCTATATGAA
GACGCCGGCC GCCGGCCGCA AGCGAGCTTT GGATATACTT

CGCGCAGCGC CAAGCCGCCG CGGTGGCTGC GGCCATTCTG
GCGCGTCGCG GTTCGGCGGC GCCACCGACG CCGGTAAGAC

GGGAAAAACG TATCGGCACC GCAACTGCCC GTGTCCTGGA
CCCTTTTTGC ATAGCCGTGG CGTTGACGGG CACAGGACCT

CGGAGATCGC TGGGCATCGC ATGCAGATGG CGGGCGATAT
GCCTCTAGCG ACCCGTAGCG TACGTCTACC GCCCGCTATA

CGAAGGACCT GGTGATTTCG TCTCGCGCGG CATGCCCGGT
GCTTCCTGGA CCACTAAAGC AGAGCGCGCC GTACGGGCCA

AGTGGCGCTG CCCTGTTGTT CCGCCTGCAG GAGCGAAGGA
TCACCGCGAC GGGACAACAA GGCGGACGTC CTCGCTTCCT

TTCAGGCGGT CGTCGCGGTC GATGCACCCC GTGACTTCGC
AAGTCCGCCA GCAGCGCCAG CTACGTGGGG CACTGAAGCG

GCTTGCAACC CGATTGGTAG AAGCCCGCGC GGCAATCGAG
CGAACGTTGG GCTAACCATC TTCGGGCGCG CCGTTAGCTC

CCAGCACGGC TCGCAGATCT TTCAAACAGT ATGCGCGATT
GGTCGTGCCG AGCGTCTAGA AAGTTTGTCA TACGCGCTAA

TTGTTCGTGC GAATGAAGGA GACCTAACGT GA
AACAAGCACG CTTACTTCCT CTGGATTGCA CT,
```

et séquences d'ADN qui à cause de la dégénération du code génétique correspondent à l'une des séquences précédentes et qui codent pour un polypeptide possédant une activité benzène oxygénase.

2. Cellule procaryote transformée à l'aide d'un ADN recombinant qui contient les segments d'ADN no I, II et III localisés sur la molécule en combinaison et qui code pour un polypeptide possédant une activité benzène oxygénase et d'un ADN vecteur, lesdits segments répondant aux formules décrites à la revendication 1.

3. Cellule selon la revendication 2, dans laquelle le procaryote est Escherichia coli.

**4.** Procédé de production du 1,2-dihydroxy-cyclohexa-3,5-diène qui comporte le traitement d'un composé aromatique par des cellules procaryotes transformées à l'aide d'un ADN recombinant contenant les segments d'ADN no I, II, et III comme définis à la revendication 1.

**5.** Procédé selon la revendication 4, dans lequel le procaryote est Escherichia coli.

**Patentansprüche**

**1.** DNA-Sequenz, die die folgenden DNA-Abschnitte mit den Nummern I, II und III in Kombination enthält, und die ein Benzoloxygenase-Aktivität aufweisendes Polypeptid codiert, wobei die Abschnitte die folgenden Formeln aufweisen:

Abschnitt Nr. I

```
ATGA  ATCAGACCGA  CACATCACCT  ATCAGGCTGC
TACT  TAGTCTGGCT  GTGTAGTGGA  TAGTCCGACG


GCAGGAGCTG  GAACACCAGC  GAGATAGAAG  CGCTCTTTGA
CGTCCTCGAC  CTTGTGGTCG  CTCTATCTTC  GCGAGAAACT


CGAGCATGCC  GGACGTATCG  ATCCGCGCAT  TTATACCGAT
GCTCGTACGG  CCTGCATAGC  TAGGCGCGTA  AATATGGCTA


GAGGATCTGT  ACCAACTCGA  ACTGGAGCGT  GTCTTCGCCC
CTCCTAGACA  TGGTTGAGCT  TGACCTCGCA  CAGAAGCGGG


GGTCCTGGCT  GCTGTTGGGG  CATGAAACCC  AGATTCGCAA
CCAGGACCGA  CGACAACCCC  GTACTTTGGG  TCTAAGCGTT


GCCGGGCGAT  TACATCACGA  CCTACATGGG  TGAAGACCCT
CGGCCCGCTA  ATGTAGTGCT  GGATGTACCC  ACTTCTGGGA


GTCGTGGTCG  TCCGGCAGAA  AGACGCCAGC  ATTGCCGTGT
CAGCACCAGC  AGGCCGTCTT  TCTGCGGTCG  TAACGGCACA


TCCTGAACCA  GTGCCGCCAC  CGTGGCATGC  GCATCTGCCG
AGGACTTGGT  CACGGCGGTG  GCACCGTACG  CGTAGACGGC


CGCGGATGCC  GGAAACGCGA  AGGCGTTCAC  TTGCAGCTAC
GCGCCTACGG  CCTTTGCGCT  TCCGCAAGTG  AACGTCGATG
```

```
CACGGGTGGG  CTTACGACAC  CGCCGGCAAT  CTTGTCAATG
GTGCCCACCC  GAATGCTGTG  GCGGCCGTTA  GAACAGTTAC


TGCCTTACGA  GGCCGAATCC  TTCGCGTGCC  TGAACAAGAA
ACGGAATGCT  CCGGCTTAGG  AAGCGCACGG  ACTTGTTCTT


GGAATGGAGC  CCGCTGAAGG  CCCGGGTAGA  AACCTACAAG
CCTTACCTCG  GGCGACTTCC  GGGCCCATCT  TTGGATGTTC


GGCCTGATTT  TCGCCAACTG  GGATGAGAAC  GCTGTAGACC
CCGGACTAAA  AGCGGTTGAC  CCTACTCTTG  CGACATCTGG


TCGACACGTA  TCTGGGCGAG  GCGAAGTTCT  ACATGGACCA
AGCTGTGCAT  AGACCCGCTC  CGCTTCAAGA  TGTACCTGGT


CATGCTCGAC  CGCACCGAGG  CCGGCACCGA  AGCGATCCCG
GTACGAGCTG  GCGTGGCTCC  GGCCGTGGCT  TCGCTAGGGC


GGCGTGCAGA  AGTGGGTCAT  TCCCTGTAAC  TGGAAATTCG
CCGCACGTCT  TCACCCAGTA  AGGGACATTG  ACCTTTAAGC


CCGCAGAGCA  GTTTTGCAGC  GACATGTACC  ATGCCGGGAC
GGCGTCTCGT  CAAAACGTCG  CTGTACATGG  TACGGCCCTG


GACCTCGCAT  CTGTCTGGCA  TCCTGGCAGG  CCTGCCAGAA
CTGGAGCGTA  GACAGACCGT  AGGACCGTCC  GGACGGTCTT


GACCTTGAAA  TGGCCGACCT  TGCTCCGCCG  ACAGTTGGCA
CTGGAACTTT  ACCGGCTGGA  ACGAGGCGGC  TGTCAACCGT


AGCAGTACCG  TGCGTCATGG  GGCGGACATG  GAAGTGGCTT
TCGTCATGGC  ACGCAGTACC  CCGCCTGTAC  CTTCACCGAA


CTATGTCGGC  GACCCCAATC  TGATGCTTGC  CATCATGGGG
GATACAGCCG  CTGGGGTTAG  ACTACGAACG  GTAGTACCCC
```

```
CCAAAGGTCA CCAGCTACTG GACCGAAGGC CCCGCGTCGG
GGTTTCCAGT GGTCGATGAC CTGGCTTCCG GGGCGCAGCC


AAAAGGCGGC CGAACGTCTG GGTAGCGTGG AGCGCGGCTC
TTTTCCGCCG GCTTGCAGAC CCATCGCACC TCGCGCCGAG


GAAACTCATG GTCGAGCACA TGACCGTCTT CCCCACGTGT
CTTTGAGTAC CAGCTCGTGT ACTGGCAGAA GGGGTGCACA


TCCTTCCTCC CAGGTATCAA TACGGTCCGG ACATTGGCAT
AGGAAGGAGG GTCCATAGTT ATGCCAGGCC TGTAACCGTA


CCGCGCGGGC CGAACGAGGT GAGGTATGGG CGTTTACGGT
GGCGCGCCCG GCTTGCTCCA CTCCATACCC GCAAATGCCA


GGTCGATGCT GATGCTCCTG ACGATATCAA GGAAGAGTTC
CCAGCTACGA CTACGAGGAC TGCTATAGTT CCTTCTCAAG


CGCGCCAGAC TGCGCACCTT CTCTCCGGTG GCGTGTTCGA
GCGCGGTCTG ACGCGTGGAA GAGAGGCCAC CGCACAAGCT


GCAGGACGAC GGGGAGAACT GGGTCGAGAT CCAGCACATC
CGTCCTGCTG CCCCTCTTGA CCCAGCTCTA GGTCGTGTAG


CTGCGAGGCC ACAAGCCGGA GCCGCCCTTT CAATGCCGAG
GACGCTCCGG TGTTCGGCCT CGGCGGGAAA GTTACGGCTC


ATGAGCATGG ACCAGACCGT CGACAACGAC CCGGTTTACC
TACTCGTACC TGGTCTGGCA GCTGTTGCTG GGCCAAATGG


CCGGGCGGAT CAGCAACAAC GTCTACAGCG AGGAAGCTGC
GGCCCGCCTA GTCGTTGTTG CAGATGTCGC TCCTTCGACG


CCGCGGGCTC TATGCCCATT GGCTGCGGAT GATGACATCC
GGCGCCCGAG ATACGGGTAA CCGACGCCTA CTACTGTAGG
```

```
CCCGACTGGG ACGCGCTGAA GGCGACACGC TGA
GGGCTGACCC TGCGCGACTT CCGCTGTGCG ACT
```

Abschnitt Nr. II

```
A TGATTGATTC AGCCAACAGA GCCGACGTCT
T ACTAACTAAG TCGGTTGTCT CGGCTGCAGA
```

```
TTCTCCGCAA GCCGGCACCC GTAGCGCCCG AACTGCAGCA
AAGAGGCGTT CGGCCGTGGG CATCGCGGGC TTGACGTCGT
```

```
CGAAGTCGAG CAGTTCTACT ATTGGGAGGC CAAGCTTCTC
GCTTCAGCTC GTCAAGATGA TAACCCTCCG GTTCGAAGAG
```

```
AACGATCGCC GCTTCGAGGA GTGGTTCGCG CTGCTCGCGG
TTGCTAGCGG CGAAGCTCCT CACCAAGCGC GACGAGCGCC
```

```
AAGACATTCA CTACTTCATG CCCATTCGCA CCACGCGGAT
TTCTGTAAGT GATGAAGTAC GGGTAAGCGT GGTGCGCCTA
```

```
CATGCGGGAC TCGCGCCTTG AATACTCAGG CTCCCGAGAG
GTACGCCCTG AGCGCGGAAC TTATGAGTCC GAGGGCTCTC
```

```
TACGCGCACT TCGATGACGA CGCCACGATG ATGAAGGGAC
ATGCGCGTGA AGCTACTGCT GCGGTGCTAC TACTTCCCTG
```

```
GCTTGCGCAA GATCACGTCC GACGTGAGCT GGTCCGAGAA
CGAACGCGTT CTAGTGCAGG CTGCACTCGA CCAGGCTCTT
```

```
CCCCGCATCG CGGACCCGGC ATCTCGTGAG CAACGTGATG
GGGGCGTAGC GCCTGGGCCG TAGAGCACTC GTTGCACTAC
```

```
ATCGTCGGCG CAGAGGCAGA AGGGGAGTAC GAAATCTCAA
TAGCAGCCGC GTCTCCGTCT TCCCCTCATG CTTTAGAGTT
```

```
GCGCCTTCAT  TGTGTACCGC  AATCGTCTGG  AGCGGCAGCT
CGCGGAAGTA  ACACATGGCG  TTAGCAGACC  TCGCCGTCGA


CGACATCTTT  GCCGGTGAGC  GTCGCGATAC  GTTGCGCCGT
GCTGTAGAAA  CGGCCACTCG  CAGCGCTATG  CAACGCGGCA


AACACGAGCG  AGGCCGGGTT  CGAGATCGTC  AATCGGACCA
TTGTGCTCGC  TCCGGCCCAA  GCTCTAGCAG  TTAGCCTGGT


TCCTGATCGA  CCAGAGCACC  ATCCTGGCCA  ATAACCTCAG
AGGACTAGCT  GGTCTCGTGG  TAGGACCGGT  TATTGGAGTC


TTTCTTCTTC  TAG
AAAGAAGAAG  ATC
```

Abschnitt Nr. III

```
ATGGCTAC  CCATGTGGCG  ATCATCGGCA  ATGGCGTGGG
TACCGATG  GGTACACCGC  TAGTAGCCGT  TACCGCACCC


TGGCTTCACG  ACCGCGCAGG  CCCTACGTGC  CGAGGGCTTC
ACCGAAGTGC  TGGCGCGTCC  GGGATGCACG  GCTCCCGAAG


GAGGGGAGAA  TCTCGCTGAT  TGGGGACGAA  CCGCATCTCC
CTCCCCTCTT  AGAGCGACTA  ACCCTGCTT  GGCGTAGAGG


CCTATGACCG  ACCATCCTTG  TCCAAGGCGG  TTCTCGACGG
GGATACTGGC  TGGTAGGAAC  AGGTTCCGCC  AAGAGCTGCC


CAGCCTTGAG  CGGCCGCCCA  TACTGGCCGA  GGCCGATTGG
GTCGGAACTC  GCCGGCGGGT  ATGACCGGCT  CCGGCTAACC


TACGGCGAGG  CCCGCATCGA  CATGCTGACC  GGCCCGGAAG
ATGCCGCTCC  GGGCGTAGCT  GTACGACTGG  CCGGGCCTTC


TCACTGCCCT  TGATGTGCAG  ACAAGGACGA  TCAGTCTGGA
AGTGACGGGA  ACTACACGTC  TGTTCCTGCT  AGTCAGACCT
```

```
TGATGGCACC  ACGCTCTCTG  CGGACGCCAT  CGTCATCGCG
ACTACCGTGG  TGCGAGAGAC  GCCTGCGGTA  GCAGTAGCGC


ACGGGCAGTC  GAGCGCGGAC  GATGGCGTTG  CCCGGCAGCC
TGCCCGTCAG  CTCGCGCCTG  CTACCGCAAC  GGGCCGTCGG


AACTGCCCGG  CGTCGTAACG  CTGCGCACCT  ACGGTGACGT
TTGACGGGCC  GCAGCATTGC  GACGCGTGGA  TGCCACTGCA


GCAGGTATTG  CGCGATAGTT  GGACTTCCGC  GACGCGGCTG
CGTCCATAAC  GCGCTATCAA  CCTGAAGGCG  CTGCGCCGAC


CTGATTGTGG  GTGGCGGATT  GATCGGCTGC  GAGGTCGCGA
GACTAACACC  CACCGCCTAA  CTAGCCGACG  CTCCAGCGCT


CGACGGCGCG  CAAGCTCGGC  CTGTCGGTCA  CGATCCTGGA
GCTGCCGCGC  GTTCGAGCCG  GACAGCCAGT  GCTAGGACCT


GGCAGGTGAT  GAACTGCTGG  TCCGAGTACT  TGGGCGGCGT
CCGTCCACTA  CTTGACGACC  AGGCTCATGA  ACCCGCCGCA


ATCGGTGCCT  GGCTGCGCGG  CCTGCTGACA  GAACTTGGTG
TAGCCACGGA  CCGACGCGCC  GGACGACTGT  CTTGAACCAC


TGCAGGTCGA  GTTGGGAACG  GGTGTCGTAG  GTTTTTCTGG
ACGTCCAGCT  CAACCCTTGC  CCACAGCATC  CAAAAAGACC


TGAGGGCCAG  CTCGAACAAG  TCATGGCCAG  CGATGGGCGC
ACTCCCGGTC  GAGCTTGTTC  AGTACCGGTC  GCTACCCGCG


AGCTTCGTAG  CCGATAGCGC  ACTCATTTGC  GTCGGCGCGG
TCGAAGCATC  GGCTATCGCG  TGAGTAAACG  CAGCCGCGCC


AGCCCGCGGA  TCAACTTGCG  CGTCAAGCGG  GCTTGGCATG
TCGGGCGCCT  AGTTGAACGC  GCAGTTCGCC  CGAACCGTAC


TGACCGCGGC  GTCATTGTCG  ATCACTGCGG  TGCGACGCTT
ACTGGCGCCG  CAGTAACAGC  TAGTGACGCC  ACGCTGCGAA
```

31

```
GCCAAAGGCT  ATTCGCCGTC  GGAGATGTGG  CCAGTTGGCG
CGGTTTCCGA  TAAGCGGCAG  CCTCTACACC  GGTCAACCGC


CTGCGGCCGG  CGGCCGGCGT  TCGCTCGAAA  CCTATATGAA
GACGCCGGCC  GCCGGCCGCA  AGCGAGCTTT  GGATATACTT


CGCGCAGCGC  CAAGCCGCCG  CGGTGGCTGC  GGCCATTCTG
GCGCGTCGCG  GTTCGGCGGC  GCCACCGACG  CCGGTAAGAC


GGGAAAAACG  TATCGGCACC  GCAACTGCCC  GTGTCCTGGA
CCCTTTTTGC  ATAGCCGTGG  CGTTGACGGG  CACAGGACCT


CGGAGATCGC  TGGGCATCGC  ATGCAGATGG  CGGGCGATAT
GCCTCTAGCG  ACCCGTAGCG  TACGTCTACC  GCCCGCTATA


CGAAGGACCT  GGTGATTTCG  TCTCGCGCGG  CATGCCCGGT
GCTTCCTGGA  CCACTAAAGC  AGAGCGCGCC  GTACGGGCCA


AGTGGCGCTG  CCCTGTTGTT  CCGCCTGCAG  GAGCGAAGGA
TCACCGCGAC  GGGACAACAA  GGCGGACGTC  CTCGCTTCCT


TTCAGGCGGT  CGTCGCGGTC  GATGCACCCC  GTGACTTCGC
AAGTCCGCCA  GCAGCGCCAG  CTACGTGGGG  CACTGAAGCG


GCTTGCAACC  CGATTGGTAG  AAGCCCGCGC  GGCAATCGAG
CGAACGTTGG  GCTAACCATC  TTCGGGCGCG  CCGTTAGCTC


CCAGCACGGC  TCGCAGATCT  TTCAAACAGT  ATGCGCGATT
GGTCGTGCCG  AGCGTCTAGA  AAGTTTGTCA  TACGCGCTAA


TTGTTCGTGC  GAATGAAGGA  GACCTAACGT  GA
AACAAGCACG  CTTACTTCCT  CTGGATTGCA  CT
```

und DNA-Sequenzen, die auf der Grundlage der Degenerierung des genetischen Codes, einer der vorstehenden DNA-Sequenzen entsprechen, und die ein Benzoloxygenase-Aktivität aufweisendes Polypeptid codieren.

**2.** Prokaryontische Zelle, transformiert mit einer rekombinanten DNA, die die darin lokalisierten folgenden DNA-Abschnitte mit den Nummern I, II und III in Kombination und eine Vektor-DNA enthält, und die ein Benzoloxygenase-Aktivität aufweisendes Polypeptid codiert, wobei die Abschnitte die in Anspruch 1 definierten Formeln aufweisen.

3. Zelle nach Anspruch 2, wobei der Prokaryont Escherichia coli ist.

4. Verfahren zur Herstellung von 1,2-Dihydroxy-cyclohexa-3,5-dien, umfassend die Behandlung einer aromatischen Verbindung mit prokaryontischen Zellen, die mit einer, die in Anspruch 1 definierten DNA-Abschnitte mit den Nummern I, II und III und III enthaltenden rekombinanten DNA transformiert sind.

5. Verfahren nach Anspruch 4, wobei der Prokaryont Escherichia coli ist.

# FIG. 1

derived from pGR115 (5.3 Kb)

derived from pGR1153 (4.2Kb)

EP 0 247 600 B1